# EUROPEAN PATENT SPECIFICATION

(11) **EP 4 203 873 B1**
(45) Date of publication and mention of the grant of the patent: **06.05.2026**
(21) Application number: 21889735.3
(22) Date of filing: 05.11.2021
(51) Int. Cl.: A61H 9/00, A61F 7/02

(54) **PORTABLE LIMB COMPRESSION SYSTEM**
TRAGBARES GLIEDMASSENKOMPRESSIONSSYSTEM
SYSTÈME PORTABLE DE COMPRESSION DE MEMBRE

(30) Priority: 05.11.2020 SG 10202011034T
(43) Date of publication of application: 05.07.2023
(73) Proprietor: National University Hospital (Singapore) Pte Ltd, Singapore 119228 (SG); National University of Singapore, Singapore 119077 (SG); Paxman Coolers Limited, Huddersfield HD8 0LE (GB)
(72) Inventor: SUNDAR, Raghav, Singapore 119228 (SG); BANDLA, Aishwarya, Singapore 117456 (SG); PAXMAN, Neil, Huddersfield HD9 4NW (GB); PAXMAN, Glenn, Holmfirth HD9 7DN (GB); MOSES, Rodney, Nottingham NH7 1FE (GB)
(74) Representative: Forresters IP LLP
(86) International application number: PCT/SG2021/050676
(87) International publication number: WO 2022/098308

(56) References cited:
- WO-A1-2007/010278
- WO-A1-2020/096737
- CN-U- 207 721 954
- US-A1- 2001 039 439
- US-A1- 2008 132 816
- US-A1- 2012 172 774
- US-A1- 2014 316 314
- US-A1- 2016 338 873
- US-A1- 2016 361 196
- US-A1- 2018 193 186
- US-A1- 2020 138 665
- US-B2- 10 463 565
- US-B2- 8 753 383

## Description

### TECHNICAL FIELD

The present disclosure relates to the application of pressure and cooling in the field of supportive care. In particular, the present disclosure relates to a compression system which may include cooling for various purposes, including the prevention of chemotherapy-induced peripheral neuropathy.

### BACKGROUND

Compression is the squeezing of a body part in a device, wrap or sleeve to apply pressure to the body part. Cooling is sometimes simultaneously carried out to effect cryocompression, which is to simultaneously apply pressure to the body part while reducing the temperature of the body part. Cryocompression has uses in various fields, including the cosmetic and medical fields. For example, cryocompression may be used to prevent and/or treat chemotherapy-induced peripheral neuropathy (CIPN). CIPN is a severe dose-limiting side-effect of several commonly used chemotherapeutic agents used in chemotherapy for cancer treatment. CIPN causes progressive and often irreversible pain/sensitivity in hands and feet and affects cancer survival rates as it may cause delay and discontinuation of chemotherapy. Overall, CIPN affects a significant number of cancer patients annually worldwide and contributes to long-term morbidity for cancer patients. CIPN also significantly increases economic burden, with healthcare costs estimated to be US$17,000 more in cancer patients with CIPN than those without CIPN. CIPN also causes patient work-loss, with a productivity loss of some 50 days with usual care.

There is an unmet and increasing clinical need for systems, devices, and methods to prevent and/or treat CIPN in cancer patients receiving chemotherapy treatment. Available treatment methods for CIPN are limited to alleviating symptoms such as paraesthesia, dysesthesia, and pain. Although several methods involving pharmacological agents have been developed, such as supplementation with Vitamin E or omega-3, none have proven effective in large-scale clinical trials.

Limb cooling during chemotherapy treatment has demonstrated a neuroprotective effect by preventing/reducing CIPN severity. Studies have shown that the extent of neuroprotection is dependent on the efficiency of limb hypothermia, i.e., the degree of cooling achieved. Studies have also shown that a patient is able to tolerate lower temperatures for longer periods of time if compression is applied concurrently when cooling.

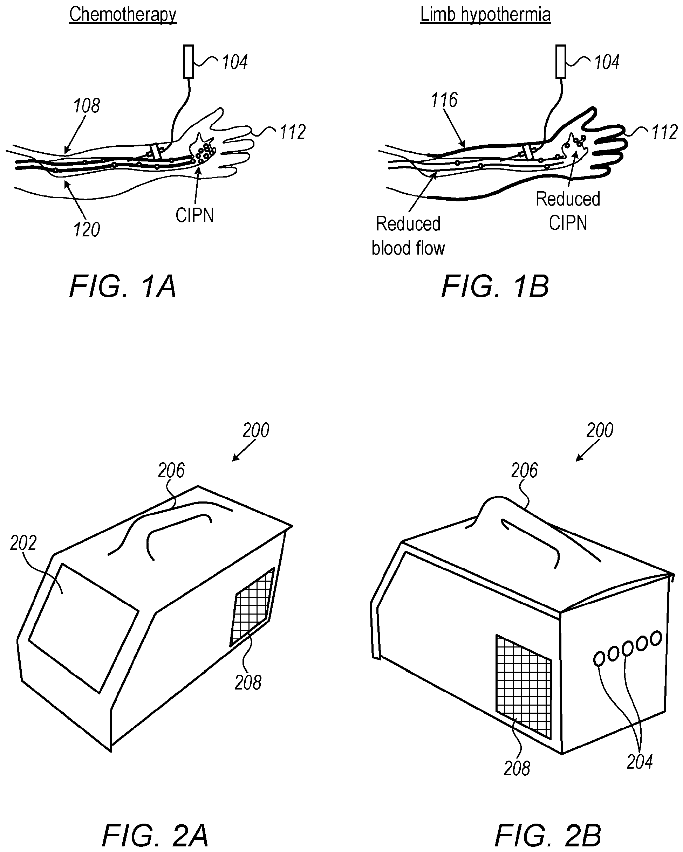

Reference is made to Figs. 1A and 1B, which illustrate a subject receiving chemotherapy treatment with and without limb hypothermia respectively. A subject may receive chemotherapy through the introduction of neurotoxic chemotherapeutics 104, like Paclitaxel, into the arm. Systematic cancer treatment with neurotoxic chemotherapeutics has been shown to cause inflammation and nerve damage in, for example, the ulnar nerve 108. This nerve damage manifests as numbness and tingling sensation in the limbs such as hand 112 and is known as CIPN. Limb hypothermia 116 prevents CIPN by causing vasoconstriction of the cooled regions such as ulnar vein 120 and reduces exposure of the region to the chemotherapeutics by reducing blood flow to the region. Limb hypothermia also reduces inflammation in the subject.

Among various cryotherapy modalities available for use, ice packs and commercially available gel packs are the most frequently used modalities. Due to risk of frost bite and subject intolerability of the temperature, studies have recommended intermittent cooling schedules of 30 minutes cooling coupled with 30 minutes of rewarming. However, such an intermittent routine may not be efficacious or, even worse, may be counter-productive owing to rebound blood flow. Furthermore, ice packs can cause extensive variations in temperature due to their phase change during melting.

Gloves were previously used frozen to administer limb cryotherapy. However, these gloves were not operator-friendly, delivered unstable cooling and caused subject discomfort which limited the period of application of cryotherapy. These gloves were eventually withdrawn from the market due to incidences of frostbite.

Other existing cooling solutions are either bulky, manpower intensive, energy inefficient, and do not cater for use in preventing CIPN in cancer patients. Existing cryotherapy/cooling apparatuses utilising continuous-controlled coolant flow use dated vapour compression technology, which is heavy/cumbersome, restricting patient-mobility, the environment of use, and consequently its range of applications. Although there are other methods for cooling, these have problems or restrictions associated. For example, cooling using the Peltier effect cannot achieve the required cooling rates whilst remaining portable. On the other hand, cooling using the Magnetocaloric effect is still at the research phase and is not market accessible.

US 2014/316314 discloses an apparatus for intermittently and sequentially compressing a body site for site specific treatment to achieve a desired temperature of the underlying tissue. The apparatus includes a first segment cooperative with a fluid chamber, the fluid chamber adapted for inflation by fluid; and a second segment cooperative with the first segment, the second segment housing a temperature sensitive material, wherein the temperature sensitive material is uniquely compartmentalized in the second segment.

US 2020/0138665 A1 discloses a compression apparatus for applying pressure to a subject, the compression apparatus comprising: a compression system; and one or more compression components connected to the compression system and secured around the subject, the one or more compression components comprising bladders capable of serving as liquid cavities for thermal fluid circulation; the compression system comprising: an air circuit configured to introduce and release air from pneumatic portions cyclically for sequential compression based on timed cycles; a liquid circuit comprising a liquid pump connected to a tank (reservoir) comprising coolant (thermal fluid), the liquid pump configured to supply the coolant to the liquid cavity by way of an output channel; and a thermoelectric cooling/heating circuit connected to the tank and configured to cool (or heat) the coolant within the tank.

US 2016/0361196 A1 discloses a thermal therapy apparatus for applying thermal treatment to a subject, the apparatus comprising: a control/compression system; and one or more pads or garments (compression components) connected to the system and secured around the subject, the components comprising channels capable of serving as liquid cavities for coolant circulation and, in certain embodiments, accommodating air for compression or purging; the system comprising: in some embodiments, an air circuit including an air compressor configured to introduce (and release) air intermittently into garment channels for compression or to purge fluid between cycles; a liquid circuit comprising a liquid pump connected to a tank (reservoir) comprising coolant, the liquid pump configured to supply the coolant to the liquid cavity by way of an output channel; and a cooling circuit (including refrigerant-based or other cooling means in various embodiments) connected to the tank/reservoir and configured to cool the coolant. In other embodiments, the liquid circuit includes recirculation paths (e.g., conduits on the return side) configured to divert some returned (used) coolant that has entered and left the liquid cavity back into the supply for temperature moderation/elevation.

There is therefore a need for an apparatus and method specifically developed to provide cryotherapy in a safe and convenient manner.

### SUMMARY OF THE DESCRIPTION

According to the present invention, there is provided a compression apparatus for applying pressure to a subject, as recited by claim 1. Further, preferable, features are presented in the dependent claims.

### SUMMARY OF THE DISCLOSURE

Implementations may include one or more of the following features.. The predetermined compression time is between 30 and 50 seconds. The predetermined decompression time is between 10 and 30 seconds. The operator-set treatment duration is between 2 and 5 hours. The air circuit may include one or more air pumps, one or more solenoid valves, and one or more pressure switches. The one or more air pumps introduces air into the air cavity and the one or more solenoid valves releases air from the air cavity. The one or more pressure switches releases air from the air cavity when the pressure within the air cavity exceeds a set pressure. The liquid cavity may be positioned between the air cavity and the subject when secured around the subject. The coolant is cooled to a temperature predetermined by an operator. The coolant has a temperature of between 6 and 24 Celsius. Implementations of the described techniques may include hardware, a method or process, or computer software on a computer-accessible medium.

### BRIEF DESCRIPTION OF THE DRAWINGS

Features, aspects, and advantages of the present disclosure will become better understood with regard to the following description and accompanying drawings in which:
Figs. 1A and 1B illustrate a subject receiving chemotherapy treatment with and without limb hypothermia respectively;
Fig. 2A is a schematic illustration of a front perspective view of an exterior of a cryocompression system, in accordance with embodiments of the present disclosure;
Fig. 2B is a schematic illustration of a rear perspective view of an exterior of a cryocompression system, in accordance with embodiments of the present disclosure;
Figs. 3A and 3B are schematic illustrations of the components of a cryocompression system, in accordance with embodiments of the present disclosure; and
Fig. 4 is a schematic flow chart illustrating a method of controlling a cryocompression process of a cryocompression component by a cryocompression system, in accordance with embodiments of the present disclosure.

With specific reference now to the drawings in detail, it is stressed that the particulars shown are by way of example and for purposes of illustrative discussion of embodiments of the disclosure. In this regard, the description taken with the drawings makes apparent to those skilled in the art how embodiments of the disclosure may be practiced.

Identical or duplicate or equivalent or similar structures, elements, or parts that appear in one or more drawings are generally labelled with the same reference numeral, optionally with an additional letter or letters to distinguish between similar entities or variants of entities, and may not be repeatedly labelled and/or described. References to previously presented elements are implied without necessarily further citing the drawing or description in which they appear.

Dimensions of components and features shown in the figures are chosen for convenience or clarity of presentation and are not necessarily shown to scale or true perspective. For convenience or clarity, some elements or structures are not shown or shown only partially and/or with different perspective or from different point of views.

### DETAILED DESCRIPTION

In the following detailed description, numerous specific details are set forth in order to provide a thorough understanding of the invention. However, it will be understood by those of ordinary skill in the art that the invention may be practiced without these specific details. In other instances, well-known methods, procedures, components, modules, units and/or circuits have not been described in detail so as not to obscure the invention.

Dimensions of components and features shown in the figures are chosen for convenience or clarity of presentation and are not necessarily shown to scale or true perspective. For convenience or clarity, some elements or structures are not shown or shown only partially and/or with different perspective or from different point of views.

Although embodiments of the invention are not limited in this regard, the terms "plurality" and "a plurality" as used herein may include, for example, "multiple" or "two or more". The terms "plurality" or "a plurality" may be used throughout the specification to describe two or more components, devices, elements, units, parameters, or the like. Unless explicitly stated, the method embodiments described herein are not constrained to a particular order or sequence. Additionally, some of the described method embodiments or elements thereof can occur or be performed simultaneously, at the same point in time, or concurrently. Unless otherwise indicated, use of the conjunction "or" as used herein is to be understood as inclusive (any or all of the stated options).

With specific reference now to the drawings in detail, it is stressed that the particulars shown are by way of example and for purposes of illustrative discussion of embodiments of the disclosure. In this regard, the description taken with the drawings makes apparent to those skilled in the art how embodiments of the disclosure may be practiced.

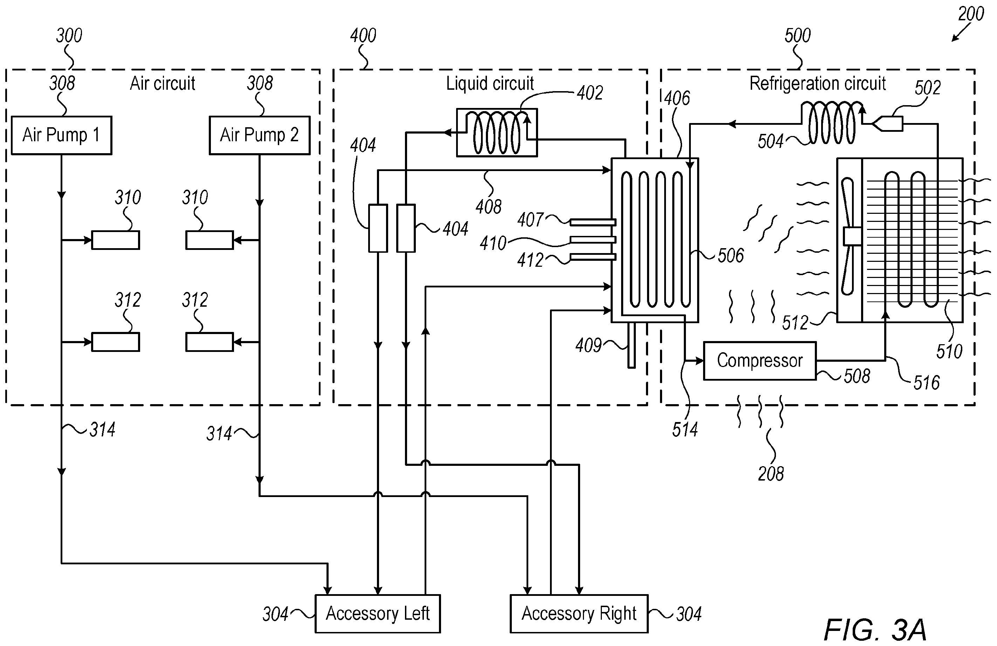

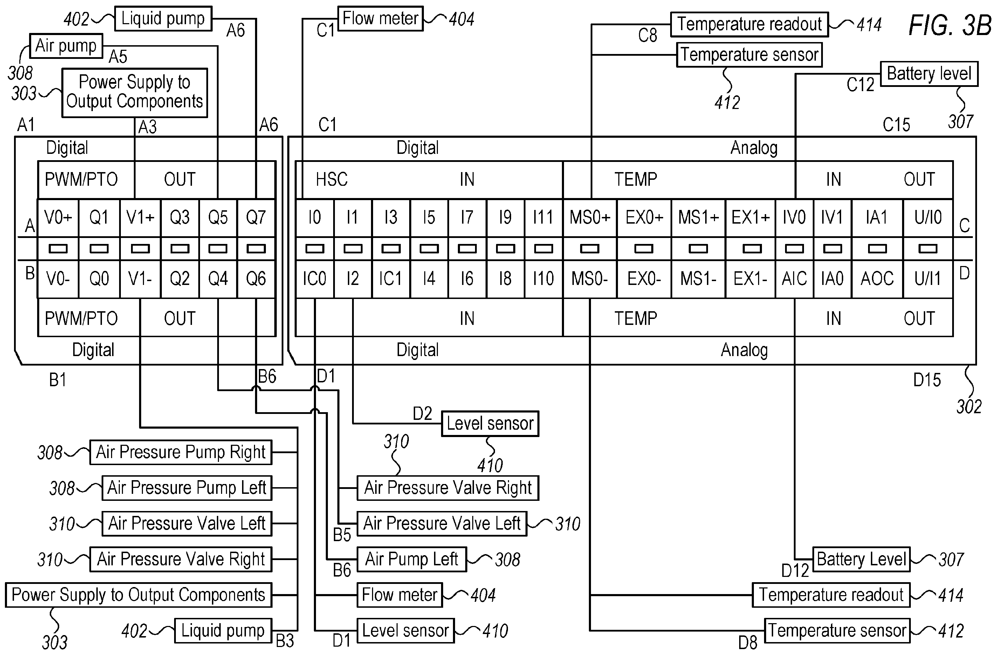

The present disclosure relates to a compression apparatus for applying pressure to a subject. The compression apparatus comprises a compression system connected to one or more compression components to be secured around a body part of the subject. In some embodiments, the compression system may be configured to only apply pressure by introducing air into the one or more compression component. In some embodiments, and as referred to below, the compression system may be a cryocompression system 200 (see Figs. 2A, 2B, 3A, and 3B) which introduces both air and coolant into separate cavities within one or more cryocompression components 304 (see Fig. 3A) to simultaneously apply pressure and cool body parts of the subject.

Reference is made to Fig. 2A which is a schematic illustration of a front perspective view of an exterior of a cryocompression system 200, while Fig. 2B is a schematic illustration of a rear perspective view of an exterior of a cryocompression system 200, in accordance with embodiments of the present disclosure. Cryocompression system 200 may comprise several features. In some embodiments, cryocompression system 200 may comprise a miniature refrigeration unit utilizing a state-of-the-art vapor compressor to provide accurate liquid temperature control and regulation (see Figs. 3A and 3B). In some embodiments, cryocompression system 200 may comprise a strong liquid pump and specially designed evaporator technology for optimal heat exchange and thermal fluid optimized dynamics (see Figs. 3A and 3B). In some embodiments, cryocompression system 200 may include a high programmable and multi-functional programmable logic controller (PLC) 302 (see Fig. 3B) system. In some embodiments, programmable logic controller 302 may be provided by Pro-face. In some embodiments, PLC 302 system may be connected to a screen 202, the PLC 302 system designed to display separate user interfaces for: healthcare professionals, subject viewing screen and service & maintenance diagnostics and data collection. In some embodiments, screen 202 may display information like temperature of coolant, level of battery, coolant level and flow rate. In some embodiments, cryocompression system 200 may be programmable for optimal user experience for users from all over the world. In some embodiments, software implemented on the PLC 302 may use a graphical programming language called ladder logic, although other graphical programming language may be employed. In some embodiments, cryocompression system 200 may comprise an input device to enable a user to input information, data, or instructions for PLC 302. Preferably, screen 202 may be a touchscreen such that screen 202 is also the input device.

According to some embodiments of the present disclosure, cryocompression system 200 may comprise multiple ports 204. In some embodiments, port 204 may be configured to receive power from an external source to power cryocompression system 200. In other embodiments, cryocompression system 200 may comprise a rechargeable, portable in-built battery supply with an external transformer (low voltage system). In some embodiments, port 204 may be an accessory port configured to connect to one or more external limb cooling accessories 304 to be secured or wrapped around one or more limbs of a subject (see Figs. 3A and 3B). In some embodiments, cryocompression system 200 may comprise independent limb controllable air intermittent compressive technology to not hinder chemotherapy infusion and two accessory ports for dual limb cooling. In some embodiments, cryocompression system 200 may be used to carry out limb cooling on either both arms or two legs of a subject and may include integrated programming to allow for a choice between upper or lower limbs.

According to some embodiments of the present disclosure, cryocompression system 200 may be housed within a portable International Organization for Standardization (ISO) 60601 considered and designed case with a single carrying handle 206. In some embodiments, cryocompression system 200 may be extremely portable and lightweight, preferably weighing at around 10 kg, allowing ease of mobility of a subject. In some embodiments, cryocompression system 200 may be able to function at ambient temperatures of around 30 °C. In some embodiments, cryocompression system 200 may be designed alongside ISO 13485 design considerations.

According to some embodiments of the present disclosure, cryocompression system 200 may comprise one or more vents 208 to allow air to pass in and out of cryocompression system 200. In some embodiments, the one or more vents 208 may be configured to allow hot air to pass out of cryocompression system 200 to prevent cryocompression system 200 from overheating. In some embodiments, the one or more vents 208 may be configured to allow cool air to pass in to cryocompression system 200 to cool cryocompression system 200.

Reference is made to Figs. 3A and 3B, which are schematic illustrations of the components of a cryocompression system 200, in accordance with embodiments of the present disclosure. In accordance with some embodiments, cryocompression system 200 comprises three circuits: an air circuit 300, a liquid circuit 400, and a refrigeration circuit 500. According to some embodiments, programmable logic controller 302 may control and provide power supply 303 to components within air circuit 300, liquid circuit 400, and refrigeration circuit 500 to effect cryocompression by one or more cryocompression components 304, or accessories 304 (see Fig. 3B). In some embodiments, programmable logic controller 302 may receive input on a current battery level 307. Preferably, cryocompression system 200 may comprise speakers which produce audible indications to alert an operator when the programmable logic controller 302 receives input that battery level 307 is below a certain percentage. In some embodiments, a beep sound may be generated when the battery level is below 40%, with a continuous sound generated when the battery level falls below 20%. In some embodiments, cryocompression components 304 comprise an air cavity and a liquid cavity, the liquid cavity positioned proximal to the skin of the subject and the air cavity positioned above the liquid cavity. The liquid cavity of cryocompression component 304 receives coolant from liquid circuit 400. Cryocompression would be performed by introducing air though air circuit 300 into the air cavity above the liquid cavity containing coolant, thus pushing the liquid cavity onto the skin of the subject. In some embodiments, the coolant used may be organic salt-based freeze depressant with pH buffer. The coolant may be of any temperature between 6 and 24 °C. Preferably, the coolant may have a density of 1.0 to 1.1 kg*m² at 20 °C to allow smooth flow within and between cryocompression system 200 and cryocompression components 304 during limb cryocompression. In some embodiments, the coolant may comprise potassium formate (CAS No: 590-29-4) (20-40%), dipotassium phosphate (CAS No: 7758-11-4) (0.5-5%) and deionised water (CAS No: 7732-18-5) (50-70%). In some embodiments, cryocompression components 304 may further comprise one or more pressure sensors (not shown) connected to programmable logic controller 302, pressure sensors providing information on the amount of pressure applied to a limb of the subject.

According to some embodiments of the present disclosure, cryocompression system 200 is connected to one or more cryocompression components 304, or accessories 304, for compression and cooling of limbs of a subject. Preferably, cryocompression system 200 is connected to two cryocompression components 304 for simultaneous compression and cooling of both arms or both legs of a subject. Cryocompression components 304 may comprise cooling mittens, gloves, covers, or other elements which may provide compression and transfer of coolant from compressor 508 to cryocompression component 304 which is worn or placed on a limb of the subject. In some embodiments, cryocompression components 304 may be activated and operational without being placed on a limb of a subject or after being placed on a limb of a subject. In some embodiments, cryocompression components 304 may also applied to other users for non-medical benefits, such as for cosmetic benefits.

In accordance with some embodiments of the present disclosure, air circuit 300 controls the air flow into the one or more cryocompression components 304 to determine the pressure applied by the one or more cryocompression components 304. Air circuit 300 of cryocompression system 200 may comprise one or more air pumps 308, one or more solenoid valves 310 (or air pressure valves 310), and one or more pressure switches 312. Programmable logic controller 302 may be electronically connected to the one or more solenoid valves 310 (or air pressure valves 310), the one or more pressure switches 312 and the one or more air pumps 308.

In accordance with some embodiments of the present disclosure, an air pump 308, a solenoid valve 310 and a pressure switch 312 may be connected to an air channel 314 connected to a cryocompression component 304. Air pump 308 may pump air into the air channel 314 and cryocompression component 304, thus increasing the amount of air in the air cavity of cryocompression component 304 and consequently the pressure applied by cryocompression component 304 on the subject. Solenoid valve 310 may release air from the air channel 314 and cryocompression component 304, thus reducing the amount of air in the air cavity of cryocompression component 304 and consequently the pressure applied by cryocompression component 304 on the subject. Pressure switch 312 may sense the pressure in air channel 314 and cryocompression component 304 and may release air from the air channel 314 and cryocompression component 304, reducing the amount of air and air pressure in the air channel 314 and cryocompression component 304, thus reducing the pressure applied by cryocompression component 304. Preferably, pressure switch 312 is mechanical, although in some embodiments, pressure switch 312 may be electronic.

In accordance with some embodiments of the present disclosure, liquid circuit 400 controls the flow of coolant into and out of the one or more cryocompression components 304. Liquid circuit 400 of cryocompression system 200 comprises a liquid pump 402and a tank 406, and may comprise one or more turbines 404 (or flow meter 404). In some embodiments, tank 406 may comprise a level sensor 410, a temperature sensor 412 and an evaporator 506. In some embodiments, tank 406 may further include filler 407 comprising an opening with a valve to fill the tank 406 with more coolant when required, as well as a drainer 409 to remove excess coolant or liquid. Programmable logic controller 302 may be electronically connected to the liquid pump 402, the one or more turbines 404 (or flow meters 404), and the components within tank 406, including the level sensor 410 and the temperature sensor 412. Level sensor 410 provides data to programmable logic controller 302 on the current coolant level, informing programmable logic controller 302 whether the coolant level is sufficient, or if the coolant level is low. Programmable logic controller 302 may then display information on the coolant level on screen 202. The one or more turbines 404 (or flow meters 404) provides data to programmable logic controller 302 that the coolant is flowing and the flow rate of the coolant. Programmable logic controller 302 may then display information on the flow rate on screen 202. Temperature sensors 412 may provide data on the current temperature of the coolant to the programmable logic controller 302. Programmable logic controller 302 may produce a temperature readout 414 displayed on screen 202.

In accordance with some embodiments of the present disclosure, coolant within tank 406 is cooled and delivered to liquid pump 402. Coolant from liquid pump 402 is pumped into the one or more cryocompression components 304. It may be pumped through a turbine 404 (or flow meter 404). A small amount of coolant is pumped back into tank 406 through an overflow channel 408 forming a short circuit of coolant flow. In some embodiments, overflow channel 408 allows for continuous flow of coolant between liquid pump 402 and tank 406 even when cryocompression components 304 are disconnected to allow agitation of coolant and prevent freeze ups or local cold spots, maintaining even temperature throughout the coolant circuit. Coolant that is pumped into the cryocompression components 304 circulates through the liquid cavity of the one or more cryocompression components 304 and exits the one or more cryocompression components 304 into the tank 406.

In accordance with some embodiments of the present disclosure, refrigeration circuit 500 cools the coolant in tank 406. Refrigeration circuit 500 may comprise an evaporator 506 within tank 406, a compressor 508, a condenser 510, a dryer 502, and a capillary 504. In some embodiments, refrigeration circuit 500 may circulate a refrigerant from compressor 508, condenser 510, dryer 502, capillary 504, through evaporator 506 within tank 406 and back to compressor 508 in a cycle. In some embodiments, compressor 508 compresses a refrigerant gas by increasing the pressure and temperature of the refrigerant gas. The pulse width modulation employed by compressor 508 may be based on the temperature of the coolant and an operator-set temperature. The hot pressurised refrigerant gas may be sent through discharge line 516 to condenser 510 and then passed through dryer 502 to remove any water before being delivered into capillary 504 where the hot pressurised refrigerant gas expands and is depressurised. In some embodiments, air may be blown over condenser 510 from outside of cryocompression system 200 to cool refrigerant circuit 500. In some embodiments, air may be pushed out of cryocompression system 200 to remove heat from refrigerant circuit 500. In some embodiments, air may be introduced into cryocompression system 200 or removed from cryocompression system 200 through vent 208. In some embodiments, the refrigerant may then be passed to evaporator 506 where it is boiled and vaporised, thus reducing the temperature of the refrigerant to sub-zero. The vaporised refrigerant may then be sucked back into the compressor 508 via suction line 514 and the cycle repeats itself.

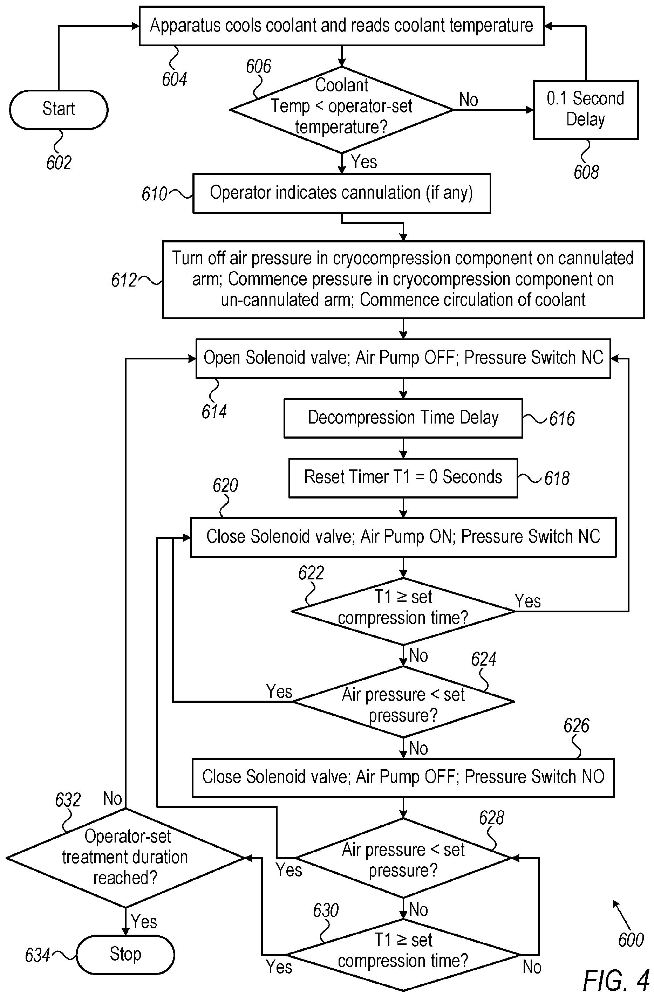

Reference is now made to Fig. 4 which is a schematic flow chart illustrating a method of controlling a cryocompression process 600 of cryocompression component 304 by cryocompression system 200, in accordance with embodiments of the present disclosure. The method of controlling a cryocompression process 600 may be implemented by programmable logic controller 302 in cryocompression system 200 which controls the different components of the cryocompression system 200. Preferably, cryocompression system 200 is powered by low voltage 24V DC power, although other suitable power sources may be used.

In accordance with some embodiments of the present disclosure, tThe method of controlling a cryocompression process 600 starts at operation 602 where cryocompression system 200 is turned on and cryocompression component 304 is turned on, after which the cryocompression system 200 cools a coolant and reads the temperature of a coolant to be circulated into the cryocompression component 304 in operation 604. Cryocompression system 200 may automatically determine in operation 606 if the coolant temperature is lower than a temperature set by the operator. Preferably, the operator is able to select from three cooling modes: low, medium, and high. Preferably, the set temperature for the low cooling mode is between 6 and 12 °C, and ideally 10 °C. Preferably, the set temperature for the medium cooling mode is between 13 and 17 °C, and ideally 15 °C. Preferably, the set temperature for the high cooling mode is between 18 and 24 °C, and ideally 20 °C. If the coolant temperature is lower than the temperature set by the operator, the cryocompression system 200 may allow the operator to indicate whether an arm of the subject is cannulated, and if so, which arm, in operation 610. If the coolant temperature is not lower than the temperature set by the operator, the cryocompression system 200 may implement a 0.1 second delay in operation 608 before implementing operation 604 again and reading the coolant temperature.

In accordance with some embodiments of the present disclosure, after the operator indicates whether an arm of the subject is cannulated in operation 610, cryocompression system 200 may turn off air pressure in the cryocompression component 304 on the cannulated arm of the subject and commence application of pressure in the cryocompression component 304 on the arm that is not cannulated in operation 612. Preferably, the pressure in the compression component is applied cyclically. Preferably, pressure is applied in predetermined cycles of 1 minute, with a predetermined compression time of between 30 and 50 seconds and a predetermined decompression time of between 10 and 30 seconds, ideally with a predetermined compression time of 50 seconds and predetermined decompression time of 10 seconds. Cryocompression system 200 may also commence circulation of coolant into cryocompression component 304 in operation 612. Preferably, the coolant is circulated into the one or more cryocompression components 304 at a flow rate of between 25 and 45 ml/sec, and ideally between 35 and 40 ml/sec.

In accordance with some embodiments of the present disclosure, programmable logic controller 302 commences application of cyclical pressure in cryocompression component 304 in operation 614. In operation 614, the programmable logic controller 302 opens solenoid valve 310 to decompress and release air from cryocompression component 304, turns off air pump 308 and inactivates pressure switch 312. The cryocompression system 200 then implements a time delay determined by the predetermined decompression time in operation 616 before resetting the timer to 0 seconds and starting the timer in operation 618 by setting the timer to 0 seconds. Once the timer is set to 0 seconds in operation 618, programmable logic controller 302 closes solenoid valve 310 and turns on air pump 308 to introduce air into cryocompression component 304 in operation 620. Pressure switch 312 remains inactivated. Once the timer determines that the predetermined compression time has been reached in operation 622, programmable logic controller 302 implements operation 614 again to repeat the pressure cycle. If the predetermined compression time has not been reached, the programmable logic controller 302 implements operation 624 in which it measures the air pressure in the cryocompression component 304. If the air pressure in the cryocompression component 304 is less than a set pressure, the programmable logic controller 302 continues operation 620 to introduce more air into the cryocompression component 304 thereby increasing the air pressure in cryocompression 304. If the air pressure in the cryocompression component 304 is not lower than set pressure, programmable logic controller 302 implements operation 626 to maintain the air pressure by closing pressure switch 312 and turning air pump 308 off. Solenoid valve 310 remains closed. Pressure switch 312 will release air if the air pressure exceeds the set pressure. Preferably, the set pressure is between 5 and 150 mmHg (0.09 and 3 PSI / 667 and 19998 Pa), and ideally is 15 mmHg (0.3 PSI / 2000 Pa).

In accordance with some embodiments of the present disclosure, after operation 626 is implemented, programmable logic controller 302 continues monitoring the air pressure in the cryocompression component 304 in operation 628. If air pressure in the cryocompression component 304 is less than a set pressure, the programmable logic controller 302 continues operation 620 to increase the air pressure to the set pressure. If the air pressure in the cryocompression component 304 does not fall below the set pressure, programmable logic controller 302 implements operation 630, which is to wait until the timer determines that the predetermined compression time has been reached. Once the timer determines that the predetermined compression time has been reached in operation 630, programmable logic controller 302 implements operation 632 to determine whether an operator-set treatment duration period has been reached. Preferably, the operator-set treatment duration is between 2 and 5 hours, and ideally 3 hours. If the operator-set treatment duration has not been reached, programmable logic controller 302 implements operation 614 again to repeat the pressure cycle. If the operator-set treatment duration has reached, the programmable logic controller 302 implements operation 634 at which cryocompression process 600 stops.

Different embodiments are disclosed herein. Features of certain embodiments may be combined with features of other embodiments; thus certain embodiments may be combinations of features of multiple embodiments. The foregoing description of the embodiments of the invention has been presented for the purposes of illustration and description. It is not intended to be exhaustive or to limit the invention to the precise form disclosed. It should be appreciated by persons skilled in the art that many modifications, variations, substitutions, changes, and equivalents are possible within the scope of the appended claims.

## Claims

1. A compression apparatus for applying pressure to a subject, the compression apparatus comprising:
a compression system (200); and
one or more compression components (304) connected to the compression system (200) and secured around the subject, the one or more compression components (304) comprising an air cavity and a liquid cavity;
the compression system (200) comprising:
an air circuit 300 configured to introduce and release air from the air cavity cyclically based on a predetermined compression time and a predetermined decompression time until an operator-set treatment duration is reached;
a liquid circuit (400) comprising a liquid pump (402) connected to a tank (406) comprising coolant, the liquid pump (402) configured to supply the coolant to the liquid cavity by way of an output channel; and
a refrigeration circuit (500) connected to the tank (406) and configured to cool the coolant within the tank (406);
wherein the liquid circuit (400) further comprises an overflow channel (408) disposed between and in fluidic communication with the output channel and the tank (406), the overflow channel (408) configured to allow at least some of the coolant leaving the liquid pump (402) to return to the tank (406) without entering the liquid cavity.

2. The compression apparatus of claim 1, wherein the predetermined compression time is between 30 and 50 seconds.

3. The compression apparatus of claim 1, wherein the predetermined decompression time is between 10 and 30 seconds.

4. The compression apparatus of claim 1, wherein the operator-set treatment duration is between 2 and 5 hours.

5. The compression apparatus of claim 1, wherein the air circuit (300) comprises one or more air pumps (308), one or more solenoid valves (310), and one or more pressure switches (312).

6. The compression apparatus of claim 5, wherein the one or more air pumps (308) is configured to introduce air into the air cavity and the one or more solenoid valves (310) is configured to release air from the air cavity.

7. The compression apparatus of claim 5, wherein the one or more pressure switches (312) are configured to release air from the air cavity when the pressure within the air cavity exceeds a set pressure

8. The compression apparatus of claim 1, wherein the liquid cavity is configured to be positioned between the air cavity and the subject when secured around the subject.

9. The compression apparatus of claim 1, wherein the refrigeration circuit (500) is configured to cool the coolant to a temperature predetermined by an operator

10. The compression apparatus of claim 1, wherein the refrigeration circuit (500) is configured to cool the coolant to a temperature of between 6°C and 24°C.

## Patentansprüche

1. Kompressionsvorrichtung zum Ausüben von Druck auf ein Subjekt, die Kompressionsvorrichtung umfassend:
ein Kompressionssystem (200); und
eine oder mehrere Kompressionskomponenten (304), die mit dem Kompressionssystem (200) verbunden und um das Subjekt herum befestigt sind, die eine oder die mehreren Kompressionskomponenten (304) umfassend einen Lufthohlraum und einen Flüssigkeitshohlraum;
das Kompressionssystem (200), umfassend:
einen Luftkreislauf (300), der konfiguriert ist, um Luft aus dem Lufthohlraum basierend auf einer vorbestimmten Kompressionszeit und einer vorbestimmten Dekompressionszeit zyklisch einzuführen und freizusetzen, bis eine von einem Bediener eingestellte Behandlungsdauer erreicht ist;
einen Flüssigkeitskreislauf (400), umfassend eine Flüssigkeitspumpe (402), die mit einem Tank (406), umfassend Kühlmittel, verbunden ist, wobei die Flüssigkeitspumpe (402) konfiguriert ist, um das Kühlmittel über einen Ausgangskanal dem Flüssigkeitshohlraum zuzuführen; und
einen Kältekreislauf (500), der mit dem Tank (406) verbunden und konfiguriert ist, um das Kühlmittel innerhalb des Tanks (406) zu kühlen;
wobei der Flüssigkeitskreislauf (400) ferner einen Überlaufkanal (408), der zwischen dem Ausgangskanal und dem Tank (406) angeordnet und mit diesen in fluider Verbindung steht, umfasst, wobei der Überlaufkanal (408) konfiguriert ist, um zu ermöglichen, dass mindestens ein Teil des Kühlmittels, das die Flüssigkeitspumpe (402) verlässt, zu dem Tank (406) zurückkehrt, ohne in den Flüssigkeitshohlraum einzutreten.

2. Kompressionsvorrichtung nach Anspruch 1, wobei die vorbestimmte Kompressionszeit zwischen 30 und 50 Sekunden liegt.

3. Kompressionsvorrichtung nach Anspruch 1, wobei die vorbestimmte Dekompressionszeit zwischen 10 und 30 Sekunden liegt.

4. Kompressionsvorrichtung nach Anspruch 1, wobei die von dem Bediener eingestellte Behandlungsdauer zwischen 2 und 5 Stunden liegt.

5. Kompressionsvorrichtung nach Anspruch 1, wobei der Luftkreislauf (300) eine oder mehrere Luftpumpen (308), ein oder mehrere Magnetventile (310) und einen oder mehrere Druckschalter (312) umfasst.

6. Kompressionsvorrichtung nach Anspruch 5, wobei die eine oder die mehreren Luftpumpen (308) konfiguriert sind, um Luft in den Lufthohlraum einzuführen, und das eine oder die mehreren Magnetventile (310) konfiguriert sind, um Luft aus dem Lufthohlraum freizusetzen.

7. Kompressionsvorrichtung nach Anspruch 5, wobei der eine oder die mehreren Druckschalter (312) konfiguriert sind, um Luft aus dem Lufthohlraum freizusetzen, wenn der Druck innerhalb des Lufthohlraums einen eingestellten Druck überschreitet.

8. Kompressionsvorrichtung nach Anspruch 1, wobei der Flüssigkeitshohlraum konfiguriert ist, um zwischen dem Lufthohlraum und dem Subjekt positioniert zu werden, wenn er um das Subjekt herum befestigt ist.

9. Kompressionsvorrichtung nach Anspruch 1, wobei der Kältekreislauf (500) konfiguriert ist, um das Kühlmittel auf eine durch einen Bediener vorbestimmte Temperatur zu kühlen.

10. Kompressionsvorrichtung nach Anspruch 1, wobei der Kältekreislauf (500) konfiguriert ist, um das Kühlmittel auf eine Temperatur zwischen 6 °C und 24 °C zu kühlen.

## Revendications

1. Appareil de compression permettant d'appliquer une pression sur un sujet, l'appareil de compression comprenant :
un système de compression (200) ; et
un ou plusieurs composants de compression (304) reliés au système de compression (200) et fixés autour du sujet, le ou les composants de compression (304) comprenant une cavité d'air et une cavité de liquide ;
le système de compression (200) comprenant :
un circuit d'air 300 configuré pour introduire et libérer de l'air de la cavité d'air de manière cyclique sur la base d'un temps de compression prédéterminé et d'un temps de décompression prédéterminé jusqu'à ce qu'une durée de traitement définie par un opérateur soit atteinte ;
un circuit de liquide (400) comprenant une pompe à liquide (402) raccordée à un réservoir (406) comprenant du liquide de refroidissement, la pompe à liquide (402) étant configurée pour alimenter la cavité de liquide en liquide de refroidissement au moyen d'un canal de sortie ; et
un circuit de réfrigération (500) raccordé au réservoir (406) et configuré pour refroidir le liquide de refroidissement à l'intérieur du réservoir (406) ;
dans lequel le circuit de liquide (400) comprend en outre un canal de débordement (408) disposé entre le canal de sortie et le réservoir (406), et en communication fluidique avec ceux-ci, le canal de débordement (408) étant conçu pour permettre à au moins une partie du liquide de refroidissement quittant la pompe à liquide (402) de retourner dans le réservoir (406) sans pénétrer dans la cavité de liquide.

2. Appareil de compression selon la revendication 1, dans lequel le temps de compression prédéterminé est compris entre 30 et 50 secondes.

3. Appareil de compression selon la revendication 1, dans lequel le temps de décompression prédéterminé est compris entre 10 et 30 secondes.

4. Appareil de compression selon la revendication 1, dans lequel la durée de traitement définie par un opérateur est comprise entre 2 et 5 heures.

5. Appareil de compression selon la revendication 1, dans lequel le circuit d'air (300) comprend une ou plusieurs pompes à air (308), une ou plusieurs électrovannes (310), et un ou plusieurs manostats (312).

6. Appareil de compression selon la revendication 5, dans lequel la ou les pompes à air (308) sont configurées pour introduire de l'air dans la cavité d'air et la ou les électrovannes (310) sont configurées pour libérer de l'air de la cavité d'air.

7. Appareil de compression selon la revendication 5, dans lequel le ou les manostats (312) sont configurés pour libérer de l'air de la cavité d'air lorsque la pression à l'intérieur de la cavité d'air dépasse une pression définie

8. Appareil de compression selon la revendication 1, dans lequel la cavité de liquide est conçue pour être positionnée entre la cavité d'air et le sujet lorsqu'elle est fixée autour du sujet.

9. Appareil de compression selon la revendication 1, dans lequel le circuit de réfrigération (500) est configuré pour refroidir le liquide de refroidissement à une température prédéterminée par un opérateur

10. Appareil de compression selon la revendication 1, dans lequel le circuit de réfrigération (500) est configuré pour refroidir le liquide de refroidissement à une température comprise entre 6 °C et 24 °C.
